# EUROPEAN PATENT APPLICATION

(11) **EP 4 032 857 A1**
(43) Date of publication of application: **27.07.2022**
(21) Application number: 20864720.6
(22) Date of filing: 11.09.2020
(51) Int. Cl.: C01D 7/12, C07C 319/20, C07C 323/58

(54) **METHOD FOR PRODUCING CONCENTRATED CARBONATE AQUEOUS SOLUTION**

(30) Priority: 17.09.2019 JP 2019168092
(71) Applicant: SUMITOMO CHEMICAL COMPANY LIMITED, Tokyo 103-6020 (JP)
(72) Inventor: NAKASUJI, Takehiro, Ichihara-shi, Chiba 299-0195 (JP); SUZUTA, Tetsuya, Niihama-shi, Ehime 792-8521 (JP); MATSUDA, Masato, Niihama-shi, Ehime 792-8521 (JP); SATO, Yuichi, Niihama-shi, Ehime 792-8521 (JP); NAKAO, Shinichi, Tokyo 178-0063 (JP)
(74) Representative: J A Kemp LLP
(86) International application number: PCT/JP2020/034580
(87) International publication number: WO 2021/054268

(57) **Abstract**

The present specification discloses a method of producing a concentrated carbonate aqueous solution. The present invention relates to a method for producing a concentrated carbonate aqueous solution, comprising a step of dewatering a hydrogen carbonate aqueous solution by means of a salt blocking membrane to prepare a concentrated hydrogen carbonate aqueous solution, wherein the concentrated hydrogen carbonate aqueous solution obtained in the above step is heated to thermally decompose the hydrogen carbonate into carbonate, carbon dioxide and water, and to evaporate water to obtain a concentrate of the carbonate aqueous solution.

## Description

### TECHNICAL FIELD

This patent application claims priority under the Paris Convention based on Japanese Patent Application No. 2019-168092 (filed on September 17, 2019), which is incorporated herein by reference in its entirety.

The present invention relates to a method for producing a concentrated carbonate aqueous solution and, more particularly, to a method for producing methionine using the method.

### BACKGROUND ART

As a method for producing methionine, which is a compound useful as a feed additive, a method is known which comprises steps of hydrolyzing 5-(2-methylthio)hydantoin with an alkali metal carbonate and water to form an aqueous solution of the alkali metal salt of methionine, adding carbon dioxide to the solution, and subjecting to separation into solid methionine and the mother liquor containing hydrogen carbonate. It is known that the filtrate containing the separated hydrogen carbonate is heated to thermally decompose the hydrogen carbonate into carbonate, carbon dioxide and water, and the water is evaporated to obtain a concentrated carbonate aqueous solution (carbonate concentration process), which is then used for hydrolysis of hydantoin (see Patent Document 1). However, in the carbonate concentration process, water evaporation by heating is not always satisfactory in terms of energy efficiency due to the high latent heat of evaporation of water.

Membrane separation technology such as RO membranes is known as a common energy-saving dewatering technology, and membrane elements are commercially available, but their application range is generally below pH 9. Therefore, the application of membranes in the carbonate concentration process (e.g. in the carbonate concentration process of the methionine production process) can be problematic as an industrial production method because the pH value of the concentrated liquid exceeds 9.

### PRIOR ART DOCUMENT

### PATENT DOCUMENT

Japanese Laid-Open Patent Publication No. 2008-506520

### SUMMARY OF THE INVENTION

### PROBLEM TO BE SOLVED BY THE INVENTION

The present invention provides a method of carbonate concentration with improved energy efficiency by the use of membrane separation, particularly a method for concentrating a carbonate aqueous solution suitable for the process of producing methionine.

### MEANS FOR SOLVING PROBLEM

The present invention encompasses the following embodiments.
1. A method for producing a concentrated carbonate aqueous solution, comprising a step of dewatering a hydrogen carbonate aqueous solution by means of a salt blocking membrane to prepare a concentrated hydrogen carbonate aqueous solution, wherein the concentrated hydrogen carbonate aqueous solution obtained in the above step is heated to thermally decompose the hydrogen carbonate into a carbonate, carbon dioxide and water, and to evaporate water to obtain a concentrate of the carbonate aqueous solution.
2. The method according to item 1, wherein the hydrogen carbonate aqueous solution subjected for dewatering by means of the salt blocking membrane is a potassium hydrogen carbonate aqueous solution or a sodium hydrogen carbonate aqueous solution.
3. The method according to item 1 or 2, wherein the hydrogen carbonate aqueous solution subjected for dewatering by the salt blocking membrane is 1 to 34% by weight hydrogen carbonate aqueous solution.
4. The method according to any one of items 1 to 3, wherein the salt blocking membrane is RO membrane or NF membrane.
5. The method according to any one of items 1 to 4, wherein the salt blocking membrane is an organic membrane.
6. The method according to any one of items 1 to 5, wherein the hydrogen carbonate aqueous solution is a hydrogen carbonate aqueous solution obtained from a methionine production process.
7. The method according to item 6, wherein the hydrogen carbonate aqueous solution is a hydrogen carbonate aqueous solution obtained by filtration of methionine.
8. A method for producing methionine which comprises,
   1) a step of reacting 3-methylmercaptopropionaldehyde, hydrogen cyanide, ammonia and carbon dioxide or ammonium carbonate to prepare 5-(2-methylmercaptoethyl) -hydantoin;
   2) a step of hydrolyzing the generated 5-(2-methylmercaptoethyl)-hydantoin with potassium carbonate and water to obtain potassium salt of methionine; and
   3) a step of introducting carbon dioxide into the generated hydrolysis reaction solution to precipitate methionine,
      said method further comprising,
   4) a step of dewatering a potassium hydrogen carbonate aqueous solution obtained by filtering methionine from a mixture of a potassium hydrogen carbonate aqueous solution and methionine generated in the above step, using a salt blocking membrane;
   5) a step of heating a membrane-concentrated potassium hydrogen carbonate aqueous solution to thermally decompose potassium hydrogen carbonate into potassium carbonate, carbon dioxide and water, and to evaporate water to obtain a concentrated aqueous solution of potassium carbonate; and
   6) a step of further subjecting the resulting concentrated potassium carbonate aqueous solution to the step 2).

### EFFECT OF THE INVENTION

The method enables recovery of a concentrated carbonate aqueous solution controlled to a predetermined concentration and by-product carbon dioxide, with greater energy efficiency than previously possible.

### MODE FOR CARRYING OUT THE INVENTION

The following is a description of a method for producing a concentrated carbonate aqueous solution, comprising a step of dewatering a hydrogen carbonate aqueous solution by means of a salt blocking membrane to prepare a concentrated hydrogen carbonate aqueous solution, wherein the concentrated hydrogen carbonate aqueous solution obtained in the above step is heated to thermally decompose the hydrogen carbonate into a carbonate, carbon dioxide and water, and to evaporate water to obtain a concentrate of the carbonate aqueous solution.

As hydrogen carbonate is an amphoteric substance (HCO₃⁻ is an acid to CO₃² and a base to H₂CO₃), the pH of the solution can be calculated from the dissociation constant, mass balance and charge balance: theoretical pH = (pKa1+pKa2)/2≈8.4. As the theoretical pH equation does not contain a concentration term, the pH is independent of the hydrogen carbonate concentration, and the concentration rate is not limited by an increase in pH, as no pH increase occurs during concentration by the membrane.

### 1) Membrane dewatering process

The concentration of the hydrogen carbonate aqueous solution to be concentrated by the salt blocking membrane is suitable for application to a concentration of 34% by weight or less, taking into account the solubility of the hydrogen carbonate at the optimum temperature for use of the membrane (usually 45°C or lower). Examples of hydrogen carbonates include sodium hydrogen carbonate or potassium hydrogen carbonate, and examples of carbonates after thermal decomposition include sodium carbonate and potassium carbonate. The pH of the hydrogen carbonate aqueous solution used for dewatering by means of the salt blocking membrane is typically below about pH 9. Concentration by means of membrane is preferably carried out at 50°C or lower, more preferably at 45°C or lower. The rate of dewatering by means of the salt blocking membrane should be 5% or more, and it is more preferable to carry out the process with 10% or more. The membrane feed pressure should be 1MPaG or higher, and it is more preferable to carry out the process with 3MPaG or higher.

Examples of salt blocking membranes include liquid separation membranes such as RO and NF membranes, which selectively permeate solvents by applying a pressure to the high concentration side that is greater than the osmotic pressure difference between the solutions existing via the membrane through which the solvent but not the solute passes. In terms of membrane structures, examples include asymmetric membranes, composite membranes and other polymeric membranes. Organic membranes are exemplified as salt blocking membranes, which can be made of, for example, but not limited to, polyamide materials such as aromatic polyamides, aliphatic polyamides and composites of these, and cellulose materials such as cellulose acetate. There is no restriction on the module format, and examples include tubular membrane modules, flat membrane modules, spiral membrane modules, and hollow fibre membrane modules. Examples of commercial elements for RO and NF membranes include SU-820FA (Toray Industries, Inc.) and CPA5-LD (Nitto Denko Corporation). Among them, SU-600, NTR-729HF, NTR-7250 and NTR-7450 can be exemplified.

### 2) Carbonate concentration process

The potassium hydrogen carbonate aqueous solution concentrated by the membrane is heated to thermally decompose the hydrogen carbonate into carbonate, carbon dioxide and water, and to evaporate water, producing a concentrated potassium carbonate aqueous solution. To facilitate the evaporation of water, the pressure is preferably 0.5MPaG or lower, and more preferably 0.1MPaG or lower. Heating and gas-liquid separation may be carried out either by heating and then leading to a gas-liquid separator or by using a distillation column. They may be concentrated to the target concentration in a single stage, or in a combination of multiple stages as in a multiple-effect can.

An example of hydrogen carbonate aqueous solutions is a hydrogen carbonate aqueous solution obtained from the methionine production process. In the following scheme, an example is shown where the hydrogen carbonate comprises an alkali metal, a typical example being potassium.

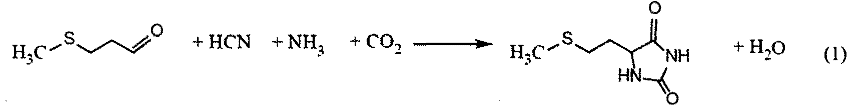

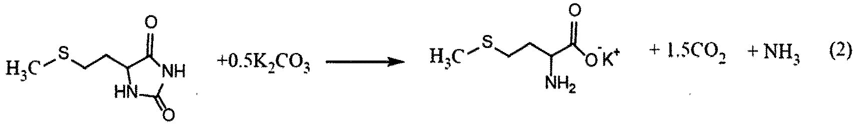

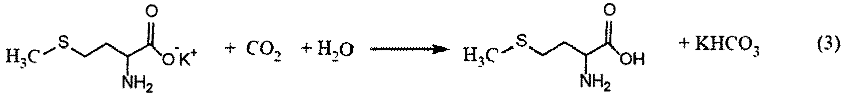

Here, after hydrolysis of the hydantoin compound with the carbonate aqueous solution as shown in formula (2), carbon dioxide gas is blown into the reaction system to neutralize the alkali as shown in formula (3), and methionine is filtered out as a solid from the mother liquor containing the carbonate aqueous solution.

The hydrogen carbonate aqueous solution obtained by filtration, after membrane concentration, undergoes decomposition by thermal treatment such as steam heating and evaporation of water, as shown in the scheme below, to result in a concentrated carbonate aqueous solution concentrated to a predetermined potassium ion concentration. The concentrated carbonate aqueous solution can be recycled to the process in formula (2) and the generated carbon dioxide to the process in formula (3).

KHCO₃ → 0.5K₂CO₃ + 0.5CO₂ + 0.5H₂O (4)

For the production process comprising the above formulas (1), (2), (3) and (4), reference may be made, for example, to US 2006/016334 and US 5770769.

Examples of the method for producing methionine, which comprises the process of producing the concentrated carbonate aqueous solution described above, include the following embodiment: a method for producing methionine which comprises,
1) a step of reacting 3-methylmercaptopropionaldehyde, hydrogen cyanide, ammonia and carbon dioxide or ammonium carbonate to prepare 5-(2-methylmercaptoethyl)-hydantoin;
2) a step of hydrolyzing the generated 5-(2-methylmercaptoethyl)-hydantoin with potassium carbonate to obtain potassium salt of methionine;
3) a step of introducting carbon dioxide into the hydrolysis reaction solution to precipitate methionine,
   said method further comprising,
4) a step of dewatering a potassium hydrogen carbonate aqueous solution obtained by filtering methionine from a mixture of a potassium hydrogen carbonate aqueous solution and methionine generated in the above step, using a salt blocking membrane;
5) a step of heating a membrane-concentrated potassium hydrogen carbonate aqueous solution to thermally decompose potassium hydrogen carbonate into potassium carbonate, carbon dioxide and water, and to evaporate water to obtain a concentrated aqueous solution of potassium carbonate; and
6) a step of further subjecting the resulting concentrated potassium carbonate aqueous solution to the step 2).

### EXAMPLES

Hereinafter, the invention will be described with reference to examples, but the present invention is not limited thereto.

### Example 1

The hydrogen carbonate aqueous solution used for membrane dewatering (hereinafter referred to as "untreated liquid") was an aqueous solution of potassium hydrogen carbonate (10% by weight) and water (90% by weight) at 25°C under atmospheric pressure, and the energy required to concentrate the potassium ions to twice the concentration of the untreated liquid by 1) Membrane dewatering process and 2) Carbonate concentration process was calculated. Although it does not contribute to energy efficiency, for the sake of calculation the flow rate of the hydrogen carbonate aqueous solution as the untreated liquid was set at 100 kg/h (potassium hydrogen carbonate: 10 kg/h, water: 90 kg/h).

### 1) Membrane dewatering process (dewatering of hydrogen carbonate aqueous solution by membrane separation)

The dewatering rate was set at 10%. As the untreated liquid has to be boosted to a certain pressure, the boosting energy was calculated with Aspen Plus (v10) and the pump efficiency was 71.27%, which is the default value of the software. The membrane feed pressure required for dewatering was increased to the osmotic pressure difference of the membrane outlet reference, where the osmotic pressure difference was highest. Osmotic pressure was calculated using the ELECNRTL model in Aspen Plus (v10).

### 2) Carbonate concentration process (thermal decomposition of hydrogen carbonate and evaporation of water)

After the concentrated hydrogen carbonate aqueous solution is lowered in pressure to atmospheric pressure, the hydrogen carbonate is thermally decomposed into carbonate, carbon dioxide and water by applying heating energy, and water is evaporated to obtain a concentrated carbonate aqueous solution. The heating energy is applied in such a way that the concentration of potassium ions in the concentrated carbonate aqueous solution reaches a predetermined concentration. Specifically, the concentrated hydrogen carbonate aqueous solution was fed into an atmospheric distillation unit (1-stage) to obtain the conditions under which the potassium ion concentration in the canned effluent reached the target concentration rate (2 times), and the heat energy given by the distillation column reboiler in achieving these conditions was taken as the required heating energy.

### (Calculation results)

The energy required for concentration by the processes 1), 2) and 3) above is 85% of that required in Comparative Example 1.

### Example 2

The same calculations were carried out using a membrane dewatering rate of 20% instead of 10% in the method of Example 1.

### (Calculation results)

The energy required for concentration is 68% of that required in Comparative Example 1.

### Example 3

In the method of Example 1, the target concentration rate of potassium ion concentration was calculated as 3 times instead of 2 times.

### (Calculation results)

The energy required for concentration is 88% of that required in Comparative Example 2.

### Example 4

The same calculations were carried out using a membrane dewatering rate of 20% instead of 10% in the method of Example 3.

### (Calculation results)

The energy required for concentration is 75% of that required for comparative example 2.

### Comparative Example 1

Thermal decomposition and concentration of hydrogen carbonate aqueous solution are conducted without carrying out membrane concentration to double the potassium ion concentration of the untreated liquid.

### Comparative Example 2

Thermal decomposition and concentration of hydrogen carbonate aqueous solution are conducted without carrying out membrane concentration to concentrate the potassium ion concentration to three times that of the untreated liquid.

The results of Examples 1 to 4 and Comparative Examples 1 and 2 are summarized in Table 1.

**[Table 1]**

| | Membrane dewatering rate | Pressure boost energy | Heating energy | Total energy (concentration energy) | Energy consumption rate |
|---|---|---|---|---|---|
| | % | GJ/h | GJ/h | GJ/h | % |
| (a)Potassium ion concentration rate : 2 times | | | | | |
| Comparative Example 1 | 0 | 0.000 | 0.140 | 0.140 | 100 |
| Example 1 | 10 | 0.002 | 0.117 | 0.119 | 85 |
| Example 2 | 20 | 0.002 | 0.094 | 0.096 | 68 |
| (b)Potassium ion concentration rate: 3 times | | | | | |
| Comparative Example 2 | 0 | 0.000 | 0.178 | 0.178 | 100 |
| Example 3 | 10 | 0.002 | 0.155 | 0.157 | 88 |
| Example 4 | 20 | 0.002 | 0.132 | 0.134 | 75 |

It has been shown that the present method is more energy efficient than the conventional method.

### INDUSTRIAL APPLICABILITY

A concentrated carbonate aqueous solution can be obtained efficiently.

## Claims

1. A method for producing a concentrated carbonate aqueous solution, comprising a step of dewatering a hydrogen carbonate aqueous solution by means of a salt blocking membrane to prepare a concentrated hydrogen carbonate aqueous solution, wherein the concentrated hydrogen carbonate aqueous solution obtained in the above step is heated to thermally decompose the hydrogen carbonate into a carbonate, carbon dioxide and water, and to evaporate water to obtain a concentrate of the carbonate aqueous solution.

2. The method according to claim 1, wherein the hydrogen carbonate aqueous solution subjected for dewatering by means of the salt blocking membrane is a potassium hydrogen carbonate aqueous solution or a sodium hydrogen carbonate aqueous solution.

3. The method according to claim 1 or 2, wherein the hydrogen carbonate aqueous solution subjected for dewatering by the salt blocking membrane is 1 to 34% by weight hydrogen carbonate aqueous solution.

4. The method according to any one of claims 1 to 3, wherein the salt blocking membrane is RO membrane or NF membrane.

5. The method according to any one of claims 1 to 4, wherein the salt blocking membrane is an organic membrane.

6. The method according to any one of claims 1 to 5, wherein the hydrogen carbonate aqueous solution is a hydrogen carbonate aqueous solution obtained from a methionine production process.

7. The method according to claim 6, wherein the hydrogen carbonate aqueous solution is a hydrogen carbonate aqueous solution obtained by filtration of methionine.

8. A method for producing methionine which comprises,
1) a step of reacting 3-methylmercaptopropionaldehyde, hydrogen cyanide, ammonia and carbon dioxide or ammonium carbonate to prepare 5-(2-methylmercaptoethyl)-hydantoin;
2) a step of hydrolyzing the generated 5-(2-methylmercaptoethyl)-hydantoin with potassium carbonate and water to obtain potassium salt of methionine; and
3) a step of introducting carbon dioxide into the generated hydrolysis reaction solution to precipitate methionine,
said method further comprising,
4) a step of dewatering a potassium hydrogen carbonate aqueous solution obtained by filtering methionine from a mixture of a potassium hydrogen carbonate aqueous solution and methionine generated in the above step, using a salt blocking membrane;
5) a step of heating a membrane-concentrated potassium hydrogen carbonate aqueous solution to thermally decompose potassium hydrogen carbonate into potassium carbonate, carbon dioxide and water, and to evaporate water to obtain a concentrated aqueous solution of potassium carbonate; and
6) a step of further subjecting the resulting concentrated potassium carbonate aqueous solution to the step 2).
